# EUROPEAN PATENT SPECIFICATION

(11) **EP 2 161 576 B1**
(45) Date of publication and mention of the grant of the patent: **04.01.2012**
(21) Application number: 09011204.6
(22) Date of filing: 01.09.2009
(51) Int. Cl.: G01N 33/53, G01N 33/569

(54) **Method of predicting risk of acquiring influenza**
Verfahren zur Vorhersage des Risikos einer Infizierung mit Influenza
Procédé de prédiction du risque d'acquisition de la grippe

(30) Priority: 01.09.2008 JP 2008223385
(43) Date of publication of application: 10.03.2010
(73) Proprietor: FUJIFILM Corporation, Tokyo 106-0031 (JP); The University of Tokushima, Tokushima-shi, Tokushima 770-8501 (JP)
(72) Inventor: Kido, Hiroshi, Tokushima-shi, Tokushima 770-8503 (JP); Terashima, Kaoru, Ashigarakami-gun, Kanagawa 258-8577 (JP)
(74) Representative: HOFFMANN EITLE

(56) References cited:
- WO-A1-03/086453
- WO-A1-2005/095974
- VIKERFORS T ET AL: "Diagnosis of influenza A virus infections by detection of specific immunoglobulins M, A, and G in serum." JOURNAL OF CLINICAL MICROBIOLOGY MAR 1989, vol. 27, no. 3, March 1989 (1989-03), pages 453-458, XP002555215 ISSN: 0095-1137
- ROTHBARTH P H ET AL: "Influenza virus serology--a comparative study." JOURNAL OF VIROLOGICAL METHODS MAR 1999, vol. 78, no. 1-2, March 1999 (1999-03), pages 163-169, XP002555216 ISSN: 0166-0934

## Description

### Technical Field

The present invention relates to a novel method for predicting the risk of acquiring influenza, which is characterized by determining each individual's risk of acquiring influenza. This method involves determining mucosal defense function of the respiratory tract, and specifically, the nasal cavity, which influenza viruses first infect, based on the level of secretory IgA (sIgA); that is, a typical host defense substance. The present invention relates to a novel method wherein nasal washings or bronchial washings is collected, and currently inevitable variation in data due to differences in the degree of washing among individuals is collected, so that airway protective ability is measured and the risk of influenza infection is objectively diagnosed.

### Background Art

High-pathogenic bird influenza has spread throughout the world to a degree such that eradication thereof is impossible. Super-flu (influenza) threats are increasing today. Countermeasures against super-flu must be taken urgently. Existing influenza vaccines are administered via subcutaneous (intramuscular) injection, so that antigen-specific IgG antibodies (that will have no effects when a new type of influenza appears) are induced in blood. However, sites at which influenza viruses infect and proliferate are mucosal epithelia of the respiratory tract or the nasal cavity, which are different from blood into which antibodies are induced. Hence, antibodies produced with the use of existing vaccines cannot act on influenza viruses (Hiroshi Kido et al., Transnasal Influenza Vaccine-Lung Surfactant and Mucosal Immunity-Pediatrics 48 (12), 1837-1844, 2007; Shinichi Tamura, Present situation of influenza vaccine development, Japanese Journal of Clinical Medicine (Nippon Rinsho) 61(11), 1993-2000, 2003; Shedlock D, Shen H, Requirement of CD4 T Cell Help in Generating Functional CD8 T Cell Memory. Science 300, 337-339, 2003). Antibodies in blood function effectively only when flu worsens to develop pneumonia, but during this time, the infection spreads. Conventionally, an anti-influenza antibody is determined based only on the antibody titer in blood, by which effects of preventing pneumonia (influenza becoming severe) can be determined, but the determination of risk of infection was difficult.

### Disclosure of the Invention

An object to be achieved by the present invention is to provide a method for predicting the risk of acquiring influenza, which is characterized by low price, low invasiveness, and applicability to total automation.

As a result of intensive studies to achieve the above object, the present inventors have discovered that each individual's risk of acquiring (infection with) influenza can be precisely determined based on the level of anti-influenza IgA antibody in nasal (mucosal) washings or airway washings that viruses first infect. Thus, the present inventors have completed the present invention. In addition, IgA antibody having high cross-immunity (i.e., IgA antibody can be effective even when a new type of influenza appears) is secreted from the respiratory tract mucosa or nasal mucosa that viruses infect, and then functions as a major factor for defense against infection and spontaneous cure.

Thus, the present provides the followings:
(1) A method for predicting the risk of acquiring influenza, which comprises measuring the ratio of anti-influenza IgA to the total IgA in a specimen collected from a subject.
(2) The method according to (1), wherein when the ratio of anti-influenza IgA to the total IgA is equal to or less than a first reference value, the risk of acquiring influenza is predicted to be high, and when the ratio of anti-influenza IgA to the total IgA is equal to or more than a second reference value, the risk of acquiring influenza is predicted to be low.
(3) The method according to (1) or (2), wherein when the ratio of anti-influenza IgA to the total IgA is equal to or less than a predetermined reference value of 2.0% or less, the risk of acquiring influenza is predicted to be high, and when the ratio of anti-influenza IgA to the total IgA is equal to or more than a predetermined reference value of 4.0% or more, the risk of acquiring influenza is predicted to be low.
(4) The method according to any one of (1) to (3), wherein the specimen is a body fluid specimen collected from head and neck mucosa.
(5) The method according to any one of (1) to (4), wherein anti-influenza IgA in a specimen collected from a subject is measured by causing the specimen to come into contact with an influenza antigen or an influenza antigen epitope which was immobilized on a solid-phase support.
(6) The method according to any one of (1) to (5), which comprises (a) immobilizing influenza antigen or influenza antigen epitope on a solid-phase support, (b) causing a specimen suspected of containing anti-influenza antibody to come into contact with the solid-phase support, (c) removing an unreacted portion of the specimen, (d) causing labeled antibody against the anti-influenza antibody in the specimen to come into contact with generated antigen-antibody complex composed of the influenza antigen or the influenza antigen epitope and the anti-influenza antibody, (e) removing unreacted labeled antibody, and (f) measuring the amount of labeling substance on the labeled antibody binding to the complex, so as to measure the presence or the amount of the anti-influenza antibody in the specimen.
(7) The method according to (6), wherein the labeled antibody is a labeled anti-human IgA antibody.
(8) The method according to (6) or (7), wherein the labeled antibody is an enzyme-labeled antibody or fluorescent-labeled antibody.
(9) The method according to (8), wherein the fluorescent-labeled antibody is an antibody which was labeled with a cyanine dye or a rhodamine 6G reagent.
(10) The method according to (9), wherein the cyanine dye is Cye3 or Cye5.
(11) A method for determining the necessity of administering an influenza vaccine or the degree of the risk of contact with an infected patient, wherein the risk of acquiring influenza is predicted by the method according to any one of (1) to (10), so that: when the risk of acquiring influenza is predicted to be high, it is determined that the necessity of administering an influenza vaccine is high or the risk of infection should be avoided to as great an extent as possible; or when the risk of acquiring influenza is predicted to be low, it is determined that necessity of administering an influenza vaccine is low. Further disclosed is a kit for measuring an anti-influenza antibody to perform the method according to any one of (1) to (10), which comprises (i) an immobilized antigen which is obtained by immobilizing an influenza antigen or an influenza antigen epitope on a solid-phase support; and (ii) a labeled antibody against an anti-influenza antibody.
(13) The kit for measuring an anti-influenza-specific antibody according to (12), wherein the solid-phase support is made of a plastic material, a fibrous material, or an inorganic material.
(14) The kit for measuring an anti-influenza-specific antibody according to (12) or (13), wherein the solid-phase support is in the form of polystyrene bead, polystyrene microtiter plate, glass slide, or polyester film.
(15) The kit for measuring an anti-influenza-specific antibody according to any one of (12) to (14), wherein the influenza antigen or the influenza antigen epitope is immobilized on the surface of the solid-phase support via an amide bond.
(16) The kit for measuring an anti-influenza-specific antibody according to any one of (12) to (15), wherein the labeled antibody against an anti-influenza antibody is a labeled anti-human IgA antibody.
(17) The kit for measuring an anti-influenza-specific antibody according to any one of (12) to (16), wherein the labeled antibody against an anti-influenza antibody is a fluorescent labeled anti-influenza antibody.

The present invention relates to a method for predicting the risk of influenza infection by measuring the anti-influenza IgA antibody titer in mucosal washings of the nasal cavity or airway secretions by a measurement method such as a fluorescent antibody method. The method for predicting the risk of acquiring influenza according to the present invention is inexpensive, relatively less invasive, and applicable to total automation by measurement using 96-well plastic plates or application thereof to high-throughput arrays, for example. The diagnosis of the risk of influenza infection according to the present invention makes it possible not only to perform preferential administration of limited amounts of a vaccine to persons with a high risk of infection, so as to be able to enhance safety against influenza infection throughout the society. It also makes it possible to provide a technique for objectively evaluating the degree of reduction of the risk of infection after vaccination. The risk of infection is diagnosed in advance based on the level of anti-influenza IgA antibody in the nasal cavity or the respiratory tract, so that "safety and reassurance" are provided to people, along with high degrees of medical economic effects. Also, the risk of infection is diagnosed in advance, making it possible to take preventive measures and implement rapid countermeasures against infection.

Specifically, the method for predicting the risk of acquiring influenza according to the present invention is industrially useful in the following respects.
(1) Based on data concerning the "diagnosis of the risk of influenza infection," selection of humans with high risks of infection becomes possible and effective use of limited amounts of novel influenza vaccines or influenza vaccines becomes possible, significantly contributing to society and the economy. Furthermore, the degree of reduction of the risk of infection of vaccinated persons can be objectively evaluated, making it possible to improve vaccine production, to increase awareness about and preventive measures against infection of an individual and to take rapid countermeasures against infection.
(2) Implementation of ELISA using microarrays makes it possible to rapidly diagnose many specimens from patients. Through inexpensive and rapid determination of the risk of infection, "safety and reassurance" and highly beneficial medical economic effects can be provided to people.

### Brief Description of the Drawings

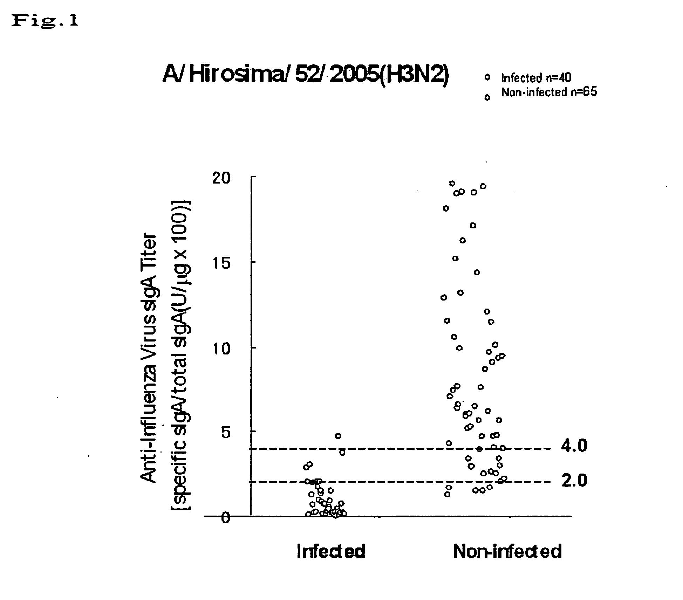

Fig. 1 shows differences in the titer of sIgA antibody reacting with the Influenza A/Hiroshima/52/2005 (H3N2) in nasal washings, as observed among influenza-infected persons and uninfected persons before infection and immediately after infection. Fig. 1 also shows the diagnosis of the risk of infection using the results.

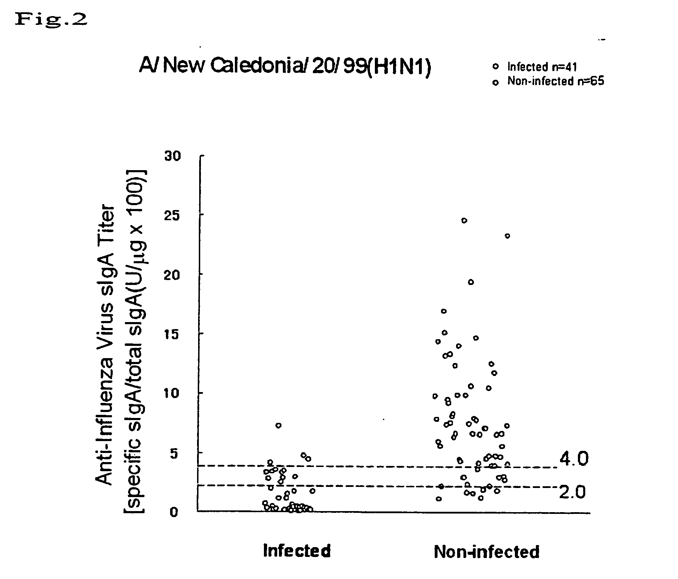

Fig. 2 shows differences in the titer of sIgA antibody reacting with the Influenza A/New Caledonia/20/99 (H1N1) in nasal washings, as observed among influenza-infected persons and uninfected persons before infection and immediately after infection. Fig. 2 also shows the diagnosis of the risk of infection using the results.

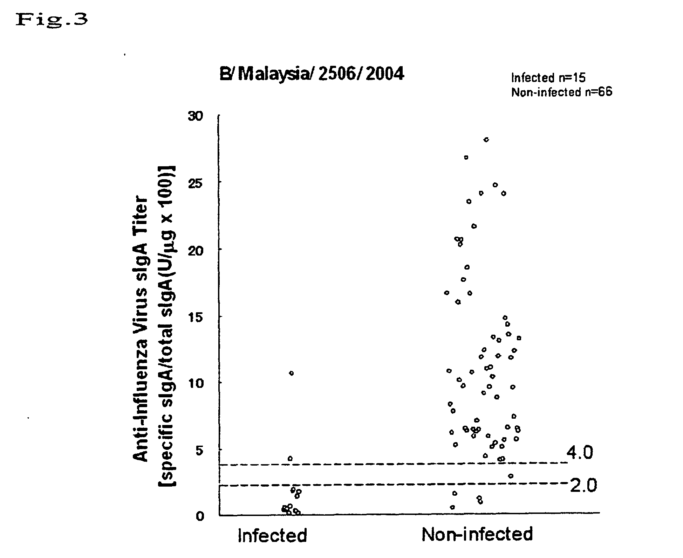

Fig. 3 shows differences in the titer of sIgA antibody reacting with the Influenza B/Malaysia/2506/2004 in nasal washings, as observed among influenza-infected persons and uninfected persons before infection and immediately after infection. Fig. 3 also shows the diagnosis of the risk of infection using the results.

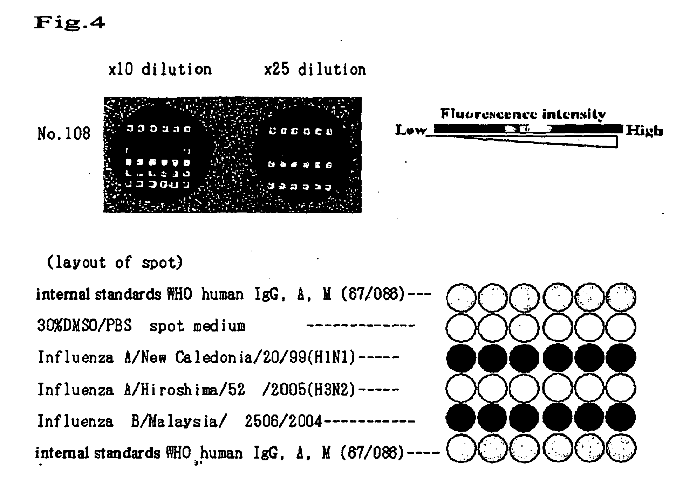

Fig. 4 shows the results of quantitative determination of anti-influenza sIgA antibody using carboxylated diamond like carbon protein arrays.

### Preferred Embodiments of the Invention

Hereafter, the present invention is described in greater detail.

The present invention is based on the discovery that the levels of local anti-influenza antibody, and particularly secretory anti-influenza IgA (sIgA) antibody in human respiratory tract mucosa that influenza viruses first infect is an important factor in determining an individual's risk of infection. In the case of mucosal washings of the nasal cavity, which can be easily collected as specimens, data are varied due to differences in specimen quality, such that some individuals have dry nasal cavities and some have wet nasal cavities. In view of such data variation, the present invention makes it possible to determine correctly the risk of infection. The method of the present invention comprises evaluating the ratio (obtained using a total sIgA level as denominator and the level of antibody sIgA specific to influenza virus as numerator) of anti-influenza IgA to the total IgA in a specimen, as an index. In addition, examples of typical strains that currently cause infection throughout the world include the following three strains, the Influenza A H3N2 strain, the Influenza A H1N1 strain, and the Influenza B strain. Other typical examples of the same (regarding which concern exists that they could cause the onset of a new type of influenza) include Influenza H5 strains such as H5N1 strain, and the H7 strains. The levels of sIgA antibodies specific to these viral strains are measured.

Specifically, before an influenza epidemic season, the level of sIgA (in nasal discharge) against the anti-Influenza A H3N2 strain, the Influenza A H1N1 strain, or the Influenza B strain is measured, and then the risk of influenza infection is diagnosed. The present invention is based on the finding that when antibody titers in mucosal washings of the nasal cavities of humans infected with influenza are measured within 48 hours during which no antibody induction has taken place yet in respiratory tract mucosa, the measured antibody titers are significantly different from the antibody titers in humans not infected with influenza. In this case, differences in the sIgA antibody titers in mucosal washings of the nasal cavities between influenza-infected persons and uninfected persons are clearer than differences in the anti-influenza IgG antibody titers in blood between influenza-infected persons and uninfected persons. This suggests that risk diagnosis is possible. Values indicating the risk of infection obtained from mucosal washings of the nasal cavities vary depending on different influenza strains.

Representative examples are as follows. In the case of the Influenza A/Hiroshima/52/2005(H3N2) strain, an antigen-specific anti-influenza sIgA antibody titer [Specific sIgA (U/mL)/Total sIgA (µg/mL) × 100] of 2.0 or less resulted in a probability of infection of 92.5% or higher, the same ranging from 2.1 to 3.9 resulted in a probability of infection of 35.0 %, and the same of 4.0 or more resulted in a probability of infection of 3.2% or lower.

In the case of the Influenza A/New caledonia/20/99 (H1N1) strain, an antigen-specific anti-influenza sIgA antibody titer [Specific sIgA (U/mL)/Total sIgA (µg/mL) × 100] of 2.0 or less resulted in a probability of infection of 89.7% or higher, the same ranging from 2.1 to 3.9 resulted in a probability of infection of 54.9%, and the same of 4.0 or more resulted in a probability of infection of 13.1 % or lower.

In the case of the Influenza B/Malaysia/2506/2004 strain, an antigen-specific anti-influenza sIgA antibody titer [Specific sIgA (U/mL)/Total sIgA (µg/mL) × 100] of 2.0 or less resulted in a probability of infection of 93.7% and the same of 4.0 or more resulted in a probability of infection of 12.5% or lower.

As described above, the method for predicting the risk of acquiring influenza according to the present invention is characterized by measuring the ratio of anti-influenza IgA to the total IgA in a specimen collected from a subject (that is, anti-influenza IgA levels/total IgA level). Specifically, when the ratio of anti-influenza IgA to total IgA corresponds to a first reference value or less, the risk of acquiring influenza is predicted to be high. When the ratio of anti-influenza IgA to total IgA corresponds to a second reference value or higher, the risk of acquiring influenza can be predicted to be low.

Such a reference value can be appropriately selected depending on target types of influenza. For example, when the ratio of anti-influenza IgA to total IgA is equal to or less than a predetermined reference value of 2% or less (e.g., 1.33%, 1.15%, or 2.0%), the risk of acquiring influenza is predicted to be high. When the ratio of anti-influenza IgA to total IgA is equal to or more than a predetermined reference value of 4% or more (e.g., 4.74%, 7.22%, or 10.65%), the risk of acquiring influenza can be predicted to be low.

Types of specimens to be used in the present invention are not particularly limited. Preferably, body fluids collected from head and neck mucosa can be used as specimens. Further preferably, specimens collected from respiratory tract mucosa and particularly preferably, specimens collected from nasal mucosa can be used.

In the present invention, anti-influenza IgA in a specimen collected from a subject can be measured by causing the specimen to come into contact with influenza antigen or influenza antigen epitope which was immobilized on a solid-phase support.

Types of solid-phase supports are not particularly limited, as long as influenza antigen or influenza antigen epitope can be immobilized thereon and then an antigen-antibody reaction can be performed. Preferably, a support made of a plastic material, a fibrous material, or an inorganic material can be used. Examples of the form of such support include beads, fine particles, membranes, and plates. Further specifically, polystyrene bead, polystyrene microtiter plate, glass slide, or polyester film can be used as a solid-phase support.

Types of influenza antigen to be used in the present invention are not particularly limited, and any influenza antigen can be used. Influenza A that includes subtypes comprising various combinations of H1 to H15 and N1 to N9, and the other Influenza B and Influenza C are used in the present invention. Examples of epidemic subtypes in recent years include the Influenza A H3N2 strain, the Influenza A H1N1 strain, and the Influenza B strain. In the case of high-pathogenic bird flu (influenza), examples of the same include various subtypes of Influenza A H5 and H7. Also, not only an influenza antigen, but also an influenza antigen epitope that is a portion thereof can also be used. The term "influenza antigen epitope" refers to a peptide comprising approximately 4 to 30 and preferably approximately 4 to 20 amino acid residues from the amino acid sequence of an influenza membrane antigen subjected to sugar chain modification.

A sugar chain-modified influenza membrane antigen or an epitope thereof can be prepared by gene recombination techniques using insect cells capable of undergoing sugar chain modification and a baculovirus vector. For a virus antigen protein not subjected to sugar chain modification, DNA encoding a desired antigen is constructed, the DNA is cloned into an expression vector, and then the vector is introduced into host cells (e.g., bacteria, yeast, or mammalian cells), so that the DNA can be expressed in the host cells to produce the desired antigen. Alternatively, an influenza antigen or an epitope thereof can also be produced by chemical synthesis. For chemical synthesis of an antigen, a general solid phase peptide synthesis method known by persons skilled in the art can be used. Alternatively, an influenza antigen can be obtained from a virus itself. For example, a desired influenza antigen or an epitope thereof can be separated and collected using centrifugation, size exclusion chromatography, or the like.

Influenza antigens or influenza antigen epitopes can be immobilized on the surface of a solid-phase support via covalent bonds (e.g., amide bonds) or noncovalent bonds (e.g., hydrogen bonds or Van der Waals force).

In a preferred embodiment of the present invention, (a) influenza antigen or influenza antigen epitope is immobilized on a solid-phase support, (b) a specimen suspected of containing anti-influenza antibody is caused to come into contact with the solid-phase support, (c) an unreacted portion of the specimen is removed, (d) labeled antibody against the anti-influenza antibody in the specimen is caused to come into contact with the generated antigen-antibody complex comprising the influenza antigen or the influenza antigen epitope and the anti-influenza antibody, (e) unreacted labeled antibody is removed, and (f) the amount of labeling substances on the labeled antibody binding to the complex is measured, so that the presence or the amount of the anti-influenza antibody in the specimen can be measured.

According to the present invention, a specimen suspected of containing anti-influenza antibody is caused to come into contact with a solid-phase support on which influenza antigen or influenza antigen epitope is immobilized, so that an antigen-antibody reaction can be performed. Subsequently, labeled antibody against the anti-influenza antibody in the specimen is caused to come into contact with antigen-antibody complex generated by the above antigen-antibody reaction, which comprises the influenza antigen or the influenza antigen epitope and the anti-influenza antibody, so that an antigen-antibody reaction is performed. The above two antigen-antibody reactions can be performed under conditions so that such antigen-antibody complexes are formed. Specifically, the antigen-antibody reactions can be performed under conditions of optimal time, temperature, and pH so that antibody can bind to its epitope. As an example of such conditions, reactions are performed using a solution with approximately pH7 to approximately pH8.5 at approximately 20°C to approximately 42°C, preferably approximately 20°C to approximately 38°C (e.g., room temperature) for approximately 1 minute to approximately 24 hours and preferably approximately 10 minutes to approximately 10 hours.

As a labeled antibody, an enzyme-labeled or fluorescent substance-labeled anti-human IgA antibody can be used. As an enzyme, alkaline phosphatase, peroxidase, or the like can be used. As a fluorescent substance, a cyanine dye (e.g., Cye3 or Cye5), a rhodamine 6G reagent, or the like can be used.

According to the present invention, the risk of acquiring influenza is predicted by the above methods. When the risk of acquiring influenza is predicted to be high, it is determined that necessity of administering an influenza vaccine is high or the risk of infection should be avoided as far as possible. When the risk of acquiring influenza is predicted to be low, it is determined that the necessity of administering an influenza vaccine is low. In this manner, necessity of administering an influenza vaccine and the degree of the risk of contact with an infected patient can be determined.

According to the present invention, an anti-influenza antibody measurement kit for predicting the risk of acquiring influenza is disclosed by combination of (i) an immobilized antigen which is obtained by immobilizing an influenza antigen or an influenza antigen epitope on a solid-phase support; and (ii) a labeled antibody against an anti-influenza antibody. This kit can further comprise an appropriate buffer, a diluent, and the like. This kit is used for performing an enzyme immunoassay (e.g., ELISA), an immunoassay using a fluorescent label or a chemiluminescent label, or an RIA method, for example.

The present invention will be explained more specifically with reference to the following examples, but the present invention is not limited to the examples.

### Examples

### (Experimental methods)

### (1) Influenza vaccine antigen

The Influenza A/Hiroshima/52/2005 (H3N2) strain, the Influenza A/New caledonia/20/99 (H1N1) strain, and the Influenza B/Malaysia/2506/2004 strain to be used as antigens in a fluorescent antibody method were vaccine (a triple vaccine as a split vaccine for subcutaneous injection) strains which were produced in 2005/2006 and 2006/2007. Each strain used as a raw material was provided by The Research Foundation for Microbial Diseases of Osaka University. As influenza vaccines to be used in the present invention, in addition to the above influenza vaccine in an ether splitting method, a β propiolactone-inactivated vaccine and a whole particle formalin-inactivated vaccine can also be used similarly. In addition to the above strains listed as representative examples, all the other influenza strains can be used herein. In addition, an example of an antigen to be used herein is a viral antigen with purity of approximately 90% or more when it is highly purified for use in vaccines. Furthermore, in applications of the present invention, antigens from bacteria, allergens such as toxoids, proteins, glycoproteins, macromolecular carbohydrates (sugar), nucleic acids, and the like, which are problematic in communicable diseases, can also be similarly used as antigens. As the value of the mass of an antigen, the actual measurement value or a value calculated based on the purity, specific activity, or molecular weight of an antigen, an antigen-antibody reaction, or the like can be used.

### (2) Preparation of nasal washing

Saline contained in a nasal spray bottle (Sun Chemical Co., Ltd., Osaka) was sprayed from the bottle tilted about 30° from the vertical direction into each nostril 10 times. A silicon catheter (MD-33105, Akita-Sumitomo Bake Co., Ltd., Akita) with a diameter of 1.7 mm was immediately inserted into each nostril. Suction was performed from each nostril for 1 minute using a suction apparatus (EP-1500, Bluecross Co., Ltd., Saitama). To collect nasal washings within catheters, 1 ml of saline was aspirated and collected in a plastic test tube. The specimens were immediately cooled with ice, subjected to 1 minute of ultrasonication, and then subjected to 10 minutes of centrifugation at 4°C and 500 × g. Thus supernatants were separated and then stored at -30°C. In this method, 0.46 ± 0.15 mL (mean ± SD) of saline in total was sprayed into both nostrils, 0.44 ± 0.37 mL (mean ± SD) of saline was collected, and the collection rate was 96%.

### (3) Quantitative determination of total influenza sIgA

Individuals show nasal dryness differently. Hence, the amounts of nasal discharge contained in sprayed saline differ depending on individuals, so that the amounts of anti-influenza sIgA contained in nasal washings are varied. Accordingly, the total sIgA level contained in a nasal washing was measured and then the ratio of the amount of anti-influenza sIgA contained therein was calculated, so that the relationship with the risk of infection was examined. In addition, secretory IgA (sIgA) comprises a serotype IgA dimeric structure to which S component is further bound. Both sIgA and IgA can be quantitatively determined with an ELISA kit for IgA.

The total sIgA level was quantitatively determined using an ELISA kit for IgA (Human IgA ELISA Quantitation Kit, BETHYL Laboratory Inc, Montgomery, TX, U.S.A.) according to the manuals. "Goat anti-human IgA-affinity purified" contained in the kit was diluted 100-fold with a coating buffer (0.05 M sodium carbonate; pH 9.6) and then 100 µL each of the diluted solution was added to each well of 96-well Nunc immunoplates (Nalgen Nunc International, NW) for ELISA, followed by 1 hour of incubation at room temperature. A TTBS wash (50 mM Tris, 0.14 M NaCl, 0.05% Tween20, pH 8.0) (300 µL) was added to each well and then removed by aspiration. This procedure was repeated 3 times and then 200 µL of a blocking buffer (50 mM Tris, 0.14 M NaCl, 1% BSA, pH 8.0) was added to each well, followed by 30 minutes of incubation at room temperature. A TTBS wash (300 µL) was added to each well and then removed by aspiration. This procedure was repeated 3 times and then a nasal washing was diluted with a sample buffer (50 mM Tris, 0.14 M NaCl, 1% BSA, 0.05% Tween20, pH 8.0). A standard preparation (from WHO) with a known concentration containing IgA, IgG, or IgM was also similarly diluted with a sample buffer. A control sample (described later) as a blank, an IgA standard preparation with a known concentration, and a specimen were separately added at 100 µL per well, followed by 1 hour of reaction at room temperature. Subsequently, 300 µL of a TTBS wash was added to each well and then removed by aspiration. This procedure was repeated 5 times, goat anti-human IgA-HRP conjugate as a secondary antibody included in the kit was diluted with a sample buffer according to the instructions, and then 100 µL of the diluted solution was added to each well, followed by 1 hour of incubation at room temperature. After reaction, 300 µL of a TTBS wash was added to each well and then removed by aspiration. This procedure was repeated 5 times. Subsequently, color reaction was performed using a TMB Microwell Peroxidase Substrate System (Kirkegaard & Perry Laboratories, Inc. Md). Finally, 100 µL of a stop solution (2 M H₂SO₄) was added and then measurement was performed at OD 450 nm using a plate reader. Based on the standard calibration curve for IgA, the value of sIgA (µg/mL) in each specimen was determined. Differences between plates and differences between days were corrected with values obtained by 500-fold dilution of pooled nasal washings. All values are expressed as differences compared with a negative control nasal washing.

### (4) Quantitative determination of anti-influenza sIgA antibody

A vaccine solution (100 µL) [1 µg (in terms of protein level) of vaccine and 100 mg of bovine serum albumin (BSA) (SIGMA) dissolved in phosphate-buffered saline (PBS)] was added to each well of 96-well Nunc immunoplates, and then solid phase reaction was performed overnight at 4°C. Subsequently, the resultant was rinsed 3 times with 300 µL of a TTBS wash, so as to remove the vaccine solutions. Subsequently, 200 µL of 50 mM Tris-HCl buffer (pH 8.0) containing 0.15 M NaCl and 1% BSA was added to each well, and then blocking reaction was performed at room temperature for 1 hour. After each well had been rinsed 3 times with a wash, the specimen was diluted to an appropriate volume using a sample buffer (50 mM Tris, 0.15 M NaCl, 1% BSA, 0.05% Tween 20, pH 8.0). IgA standard preparation of WHO was also diluted similarly with a sample buffer. A control sample (described later) as a blank and an IgA standard specimen were added at 100 µL per well, followed by 2 hours of reaction at room temperature. Next, each well was washed with 300 µL of a TTBS wash, removed by aspiration. After this procedure was repeated 5 times, a goat anti-human IgA-HRP conjugate included as a secondary antibody in a kit was diluted with a sample buffer according to the instruction. The diluted solution was added at 100 µL per well and then incubation was performed at room temperature for 1 hour. After reaction, 300 µL of a TTBS wash was added to each well and then removed by aspiration. This procedure was repeated 5 times. Next, color reaction was performed using a TMB Microwell Peroxidase Substrate System (Kirkegaard & Perry Laboratories, Inc. MD). Finally, 100 µL of a stop solution (2 M H₂SO₄) was added and then measurement was performed using a plate reader at OD 450 nm. Theoretically, it is optimal to use anti-influenza sIgA against each vaccine antigen (affinity-purified from human nasal washing) as a standard for quantitative determination. However, sampling thereof is limited or restricted. Instead of this, relative concentrations (Units) were measured using the calibration curve used for measurement of total IgA, for the sake of convenience. Therefore, the thus obtained values were expressed in the form of "Unit/mL." To correct differences between plates and differences between days, values of specimens obtained via 500-fold dilution of pooled nasal washings were used. All values are expressed as differences compared with a negative control nasal washing.

### (5) Preparation of control sample for background measurement

Nasal washings of 57 subjects were pooled and used as pooled nasal washings for correction of differences between plates and differences between days. Meanwhile, for preparation of a negative control nasal washing, a nasal washing was added to a Petri dish with a diameter of 3 cm, on which a mixed vaccine (used as an antigen) of the Influenza A/Hiroshima/52/2005 (H3N2) strain, the Influenza A/New caledonia/20/99 (H1N1) strain, and the Influenza B/Malaysia/2506/2004 strain had been immobilized, and then reaction was performed overnight at 4°C, so that antibodies were absorbed. This procedure was repeated until no antibody was detected in the nasal washing, so that a negative control nasal washing was prepared.

### (6) Quantitative determination of anti-influenza sIgA antibody using carboxylated Diamond like carbon protein array

Methods for applying carboxylated Diamond-like carbon arrays as protein chips have been reported to date (JP Patent Publication (Kokai) No. 2006-267058 A: Method for Immobilization of Protein/Peptide to Diamond Chip; JP Patent Publication (Kokai) No. 2006-267063 A: Method for Determining Allergic Disease and Kit for Determining Allergic Disease; and PCT/JP2008/000242: Method for Determining Allergic Disease). Application was performed according to these methods. Specifically, influenza vaccines were immobilized on carboxylated Diamond-like carbon arrays by the following method.

Specifically, various influenza vaccine strains, each of which had been adjusted to 1 mg/ml in terms of protein level, WHO human IgG, A, and M (67/086) 200 U/mL (NIBSC) as internal standards, and a sample solution (10 mg/ml BSA/0.05% Tween20/0.3% KCl/PBS) were each diluted 200-fold with a 30% dimethyl sulfoxide (DMSO)/PBS solution. The diluted solution was spotted onto an array using an OmniGrid^{R} Accent (Genomic Solutions-NIPPUN Techno Cluster). Next, the resultant was shileded from light and dried at 37°C for 3 hours. A mixed solution of NanoBio Bloker (Nano Bio Tech) and a BSA solution (10 mg/ml BSA, 0.1 M Glycine, 0.1% PEG/PBS) mixed at a ratio of 1 : 3 was added as a blocking solution at 10 µL per spot. The resultants were shielded from light and left to stand at 4°C overnight, so as to perform blocking. Subsequently, chips were lightly washed with MilliQ water and then washed with TTBS once for 5 minutes. The chips were washed lightly again with MilliQ and then centrifuged at 150 × g for 2 minutes, thereby draining water. Thereafter, a nasal washing diluted with a sample solution was added at 5 µL per spot. The resultants were shileded from light and caused to undergo reaction at 37°C for 1 hour. After reaction, light wasing was performed with MilliQ to remove specimens and then 5 minutes of washing was performed twice with TTBS. After light washing with MilliQ, the resultants were centrifuged at 150 × g for 2 minutes, thereby draining water. Regarding a nasal washing specimen, anti-human IgA labeled with a fluorescent dye Cy3 was added as a secondary antibody at 5 µL per spot. The resultants were shileded from light and caused to undergo reaction at 37°C for 1 hour. Subsequently, light washing was performed with MilliQ water and then two instances of washing each 5 minutes long were performed with TTBS. After light wasing was performed again with MilliQ water, centrifugation was performed at 150 × g for 2 minutes, thereby draining water. Finally, fluorescence intensity was measured at 532 nm (FUJIFILM FLA-8000).

### (Results)

### (1) Differences in the titer of sIgA antibody reacting with the Influenza A/Hiroshima/52/2005 (H3N2) strain in nasal washings, as observed among influenza-infected and uninfected persons, before infection and immediately after infection, and diagnosis of the risk of infection using the results

Fig. 1 shows the anti-Influenza A/Hiroshima/52/2005 (H3N2) sIgA antibody titers in nasal washings of: 41 volunteer patients infected with influenza (based on definitive diagnosis made by detection of viral antigens using a rapid diagnostic kit for influenza, Espline Influenza A&B-N, Fujirebio, Tokyo) during influenza seasons in 2005/2006, and 2006/2007 in Japan, such patients having visited hospitals within 48 hours after the onset of fever and from whom nasal washings could be collected; and 70 volunteers not infected with influenza, from whom nasal washings could be collected in November during each influenza season. Fig. 1 shows the results represented by antigen-specific anti-influenza sIgA antibody titers [Rate of anti-influenza virus sIgA: Specific sIgA (U/mL)/Total sIgA (µg/mL) × 100]. Whereas the average value of antigen-specific anti-influenza sIgA antibody titers of influenza-infected persons was 1.04 ± 1.10 (mean ± SD), the same for uninfected persons was 9.98 ± 8.39, showing a statistically significant difference (p < 0.01: Mann-Whitney U-test) between the two groups. Moreover, in the case of an antigen-specific anti-influenza sIgA antibody titer [Specific sIgA (U/mL)/Total sIgA (µg/mL) × 100] of 2.0 or less, a probability of infection was found to be 92.5% or higher. In the case of the same ranging from 2.1 to 3.9, a probability of infection was found to be 35.0 % and in the case of the same of 4.0 or more, a probability of infection was found to be 3.2% or lower.

### (2) Differences in the titer of sIgA antibody reacting with the Influenza A/New Caledonia/20/99(H1N1) in nasal washings, as observed among influenza-infected and uninfected persons, before infection and immediately after infection, and diagnosis of the risk of infection using the results

Fig. 2 shows anti-Influenza A/New Caledonia/20/99 (H1N1) sIgA antibody titers in nasal washings of: 41 volunteer patients infected with influenza (based on definitive diagnosis made by detection of viral antigens using a rapid diagnostic kit for influenza, Espline Influenza A&B-N, Fujirebio, Tokyo) during influenza seasons in 2005/2006, and 2006/2007 in Japan, such patients having visited hospitals within 48 hours after the onset of fever and from whom nasal washings could be collected; and 66 volunteers not infected with influenza from whom nasal washings could be collected in November during each influenza season. Fig. 2 shows the results represented by antigen-specific anti-influenza sIgA antibody titers [Rate of anti-influenza virus sIgA: Specific sIgA (U/mL)/Total sIgA (µg/mL) × 100]. Whereas the average value of antigen-specific anti-influenza sIgA antibody titers of influenza-infected persons was 1.56 ± 1.71 (mean ± SD), the same for uninfected persons was 7.96 ± 5.93, showing a statistically significant difference (*p* < 0.01: Mann-Whitney U-test) between the two groups. Moreover, in the case of an antigen-specific anti-influenza sIgA antibody titer [Specific sIgA(U/mL)/Total sIgA (µg/mL) × 100] of 2.0 or less, a probability of infection was found to be 89.7% or higher. In the case of the same ranging from 2.1 to 3.9, a probability of infection was found to be 54.9%. In the case of the same of 4.0 or more, a probability of infection was found to be 13.1 % or lower.

### (3) Differences in the titer of sIgA antibody reacting with the Influenza B/Malaysia/2506/2004 in nasal washings, as observed among influenza-infected and uninfected persons, before infection and immediately after infection, and diagnosis of the risk of infection using the results

Fig. 3 shows anti-Influenza B/Malaysia/2506/2004 sIgA antibody titers in nasal washings of: 41 volunteer patients infected with influenza (based on definitive diagnosis made by detection of viral antigens using a rapid diagnostic kit for influenza, Espline Influenza A&B-N, Fujirebio, Tokyo) during influenza seasons in 2005/2006, and 2006/2007 in Japan, such patients having visited hospitals within 48 hours after the onset of fever and from whom nasal washings could be collected; and 70 volunteers not infected with influenza, from whom nasal washings could be collected in November during each influenza season. Fig. 3 shows the results represented by antigen-specific anti-influenza sIgA antibody titers [Rate of anti-influenza virus sIgA: Specific sIgA(U/mL)/Total sIgA (µg/mL) × 100]. Whereas the average value of antigen-specific anti-influenza sIgA antibody titers of influenza-infected persons was 1.65 ± 2.72 (mean ± SD), the same for uninfected persons was 10.94 ± 6.75, showing a statistically significant difference (p < 0.01: Mann-Whitney U-test) between the two groups. Moreover, in the case of an antigen-specific anti-influenza sIgA antibody titer [Specific sIgA (U/mL)/Total sIgA (µg/mL) × 100] of 2.0 or less, a probability of infection was found to be 93.7%. In the case of the same of 4.0 or more, a probability of infection was found to be 12.5% or lower.

### (4) Quantitative determination of anti-influenza sIgA antibody using carboxylated diamond like carbon protein array

With the use of methods described in Experimental Methods, nasal washings of: 41 volunteer patients infected with influenza (based on definitive diagnosis made by detection of viral antigens using a rapid diagnostic kit for influenza, Espline Influenza A&B-N, Fujirebio, Tokyo) during influenza seasons in 2005/2006, and 2006/2007 in Japan, such patients having visited hospitals within 48 hours after the onset of fever and from whom nasal washings could be collected; and 66 volunteers not infected with influenza from whom nasal washings could be collected in November during each influenza season were used as specimens. When carboxylated diamond like carbon protein arrays were used, sIgA antibody titers of anti-Influenza A/Hiroshima/52/2005 (H3N2), anti-Influenza A/New Caledonia/20/99 (H1N1), and anti-Influenza B/Malaysia/2506/2004 were measured simultaneously. The concentration of each antibody binding to an antigen was correlated as a binding unit with the results of the above-described 96-well Nunc immunoplate assay, so that risks were diagnosed. Fig. 4 shows an example of the nasal washing of one patient as a representative example. It was demonstrated that quantitative determination of influenza-specific sIgA was possible by the use of arrays.

## Claims

1. A method for predicting the risk of acquiring influenza, which comprises measuring the ratio of anti-influenza IgA to the total IgA in a specimen collected from a subject, wherein the specimen is a body fluid specimen collected from head and neck mucosa.

2. The method according to claim 1, wherein when the ratio of anti-influenza IgA to the total IgA is equal to or less than a first reference value, the risk of acquiring influenza is predicted to be high, and when the ratio of anti-influenza IgA to the total IgA is equal to or more than a second reference value, the risk of acquiring influenza is predicted to be low.

3. The method according to claim 1 or 2, wherein when the ratio of anti-influenza IgA to the total IgA is equal to or less than a predetermined reference value of 2.0% or less, the risk of acquiring influenza is predicted to be high, and when the ratio of anti-influenza IgA to the total IgA is equal to or more than a predetermined reference value of 4.0% or more, the risk of acquiring influenza is predicted to be low.

4. The method according to any one of claims 1 to 3, wherein anti-influenza IgA in a specimen collected from a subject is measured by causing the specimen to come into contact with an influenza antigen or an influenza antigen epitope which was immobilized on a solid-phase support.

5. The method according to any one of claims 1 to 4, which comprises (a) immobilizing influenza antigen or influenza antigen epitope on a solid-phase support, (b) causing a specimen suspected of containing anti-influenza antibody to come into contact with the solid-phase support, (c) removing an unreacted portion of the specimen, (d) causing labeled antibody against the anti-influenza antibody in the specimen to come into contact with generated antigen-antibody complex composed of the influenza antigen or the influenza antigen epitope and the anti-influenza antibody, (e) removing unreacted labeled antibody, and (f) measuring the amount of labeling substance on the labeled antibody binding to the complex, so as to measure the presence or the amount of the anti-influenza antibody in the specimen.

6. The method according to claim 5, wherein the labeled antibody is a labeled anti-human IgA antibody.

7. The method according to claim 5 or 6, wherein the labeled antibody is an enzyme-labeled antibody or fluorescent-labeled antibody.

8. The method according to claim 7, wherein the fluorescent-labeled antibody is an antibody which was labeled with a cyanine dye or a rhodamine 6G reagent.

9. The method according to claim 8, wherein the cyanine dye is Cye3 or Cye5.

10. A method for determining the necessity of administering an influenza vaccine or the degree of the risk of contact with an infected patient, wherein the risk of acquiring influenza is predicted by the method according to any one of claims 1 to 9, so that: when the risk of acquiring influenza is predicted to be high, it is determined that the necessity of administering an influenza vaccine is high or the risk of infection should be avoided to as great an extent as possible; or when the risk of acquiring influenza is predicted to be low, it is determined that necessity of administering an influenza vaccine is low.

## Patentansprüche

1. Verfahren zur Prognose des Influenza-Infektionsrisikos, umfassend die Messung des Verhältnisses von Anti-Influenza-IgA zu Gesamt-IgA in einer Probe, die von einem Patienten genommen wurde, wobei die Probe eine Körperflüssigkeitsprobe ist, die aus Kopf- und Hals-Schleimhaut genommen wurde.

2. Verfahren gemäß Anspruch 1, wobei das Influenza-Infektionsrisiko als hoch prognostiziert wird, wenn das Verhältnis von Anti-Influenza-IgA zu Gesamt-IgA gleich oder kleiner als ein erster Referenzwert ist, und das Influenza-Infektionsrisiko als niedrig prognostiziert wird, wenn das Verhältnis von Anti-Influenza-IgA zu Gesamt-IgA gleich oder größer als ein zweiter Referenzwert ist.

3. Verfahren gemäß Anspruch 1 oder 2, wobei das Influenza-Infektionsrisiko als hoch prognostiziert wird, wenn das Verhältnis von Anti-Influenza-IgA zu Gesamt-IgA gleich oder kleiner als ein vorgegebener Referenzwert von 2,0 % oder weniger ist, und das Influenza-Infektionsrisiko als niedrig prognostiziert wird, wenn das Verhältnis von Anti-Influenza-IgA zu Gesamt-IgA gleich oder größer als ein vorgegebener Referenzwert von 4,0 % oder mehr ist.

4. Verfahren gemäß einem der Ansprüche 1 bis 3, wobei Anti-Influenza-IgA in einer von einem Patienten genommenen Probe gemessen wird, indem die Probe mit einem Influenza-Antigen oder einem Influenza-Antigen-Epitop in Kontakt gebracht wird, das an einem Festphasen-Träger immobilisiert wurde.

5. Verfahren gemäß einem der Ansprüche 1 bis 4, umfassend (a) Immobilisierung von Influenza-Antigen oder Influenza-Antigen-Epitop an einem Festphasen-Träger, (b) in Kontakt bringen einer Probe, von der vermutet wird, dass sie Anti-Influenza-Antikörper enthält, mit dem Festphasen-Träger, (c) Entfernen eines ungebundenen Teils der Probe (d) in Kontakt bringen von markiertem Antikörper gegen den Anti-Influenza-Antikörper in der Probe mit dem erzeugten Antigen-Antikörper-Komplex, zusammengesetzt aus dem Influenza-Antigen oder dem Influenza-Antigen-Epitop und dem Anti-Influenza-Antikörper, (e) Entfernen von ungebundenem markiertem Antikörper und (f) Messen der Menge an markierter Substanz auf dem markierten Antikörper, der an den Komplex bindet, um so die Anwesenheit oder die Menge an Anti-Influenza-Antikörper in der Probe zu messen.

6. Verfahren gemäß Anspruch 5, wobei der markierte Antikörper ein markierter Anti-Human-IgA-Antikörper ist.

7. Verfahren gemäß Anspruch 5 oder 6, wobei der markierte Antikörper ein Enzym-markierter Antikörper oder Fluoreszenz-markierter Antikörper ist.

8. Verfahren gemäß Anspruch 7, wobei der Fluoreszenzmarkierte Antikörper ein Antikörper ist, der mit einem Cyaninfarbstoff oder einem Rhodamin-6G-Reagenz markiert ist.

9. Verfahren gemäß Anspruch 8, wobei der Cyaninfarbstoff Cye3 oder Cye5 ist.

10. Verfahren zur Bestimmung der Notwendigkeit, eine Influenza-Vaccine zu verabreichen, oder des Risikogrades bei Kontakt mit einem infizierten Patienten, wobei das Influenza-Infektionsrisiko mittels des Verfahrens gemäß einem der Ansprüche 1 bis 9 prognostiziert wird, so dass bestimmt wird, dass die Notwendigkeit eine Influenza-Vaccine zu verabreichen hoch ist oder das Infektionsrisiko so weit wie möglich vermieden werden sollte, wenn das Influenza-Infektionsrisiko als hoch prognostiziert wird oder bestimmt wird, dass die Notwendigkeit eine Influenza-Vaccine zu verabreichen niedrig ist, wenn das Influenza-Infektionsrisiko als niedrig prognostiziert wird.

## Revendications

1. Procédé pour prédire le risque d'attraper la grippe, qui comprend la mesure du rapport de l'IgA antigrippale à l'IgA totale dans un échantillon collecté auprès d'un sujet, dans lequel l'échantillon est un échantillon de fluide corporel collecté à partir des muqueuses de la tête et du cou.

2. Procédé selon la revendication 1, dans lequel, quand le rapport de l'IgA antigrippale à l'IgA totale est égal ou inférieur à une première valeur de référence, le risque d'attraper la grippe est prédit comme étant élevé, et quand le rapport de l'IgA antigrippale à l'IgA totale est égal ou supérieur à une deuxième valeur de référence, le risque d'attraper la grippe est prédit comme étant faible.

3. Procédé selon la revendication 1 ou 2, dans lequel, quand le rapport de l'IgA antigrippale à l'IgA totale est égal ou inférieur à une valeur de référence prédéterminée de 2,0 % ou moins, le risque d'attraper la grippe est prédit comme étant élevé, et quand le rapport de l'IgA antigrippale à l'IgA totale est égal ou supérieur à une valeur de référence prédéterminée de 4,0 % ou plus, le risque d'attraper la grippe est prédit comme étant faible.

4. Procédé selon l'une quelconque des revendications 1 à 3, dans lequel l'IgA antigrippale dans un échantillon collecté auprès d'un sujet est mesurée par l'opération consistant à mettre l'échantillon en contact avec un antigène grippal ou un épitope d'antigène grippal qui a été immobilisé sur un support en phase solide.

5. Procédé selon l'une quelconque des revendications 1 à 4, qui comprend (a) l'immobilisation d'antigène grippal ou d'épitope d'antigène grippal sur un support en phase solide, (b) la mise en contact d'un échantillon suspecté de contenir un anticorps antigrippal avec le support en phase solide, (c) le retrait de la partie de l'échantillon n'ayant pas réagi, (d) la mise en contact d'un anticorps marqué, dirigé contre l'anticorps antigrippal dans l'échantillon, avec le complexe antigène-anticorps généré, composé de l'antigène grippal ou de l'épitope d'antigène grippal et de l'anticorps antigrippal, (e) le retrait de l'anticorps marqué n'ayant pas réagi, et (f) la mesure de la quantité de substance de marquage sur l'anticorps marqué se liant au complexe, de façon à mesurer la présence ou la quantité de l'anticorps antigrippal dans l'échantillon.

6. Procédé selon la revendication 5, dans lequel l'anticorps marqué est un anticorps anti-IgA humaine marqué.

7. Procédé selon la revendication 5 ou 6, dans lequel l'anticorps marqué est un anticorps marqué par une enzyme ou un anticorps marqué par fluorescence.

8. Procédé selon la revendication 7, dans lequel l'anticorps marqué par fluorescence est un anticorps qui a été marqué avec un colorant cyanine ou un réactif rhodamine 6G.

9. Procédé selon la revendication 8, dans lequel le colorant cyanine est Cye3 ou Cye5.

10. Procédé pour déterminer la nécessité d'administrer un vaccin contre la grippe ou le degré du risque de contact avec un patient infecté, dans lequel le risque d'attraper la grippe est prédit par le procédé selon l'une quelconque des revendications 1 à 9, de façon que : lorsque le risque d'attraper la grippe est prédit comme étant élevé, il est déterminé que la nécessité d'administrer un vaccin contre la grippe est élevée ou que le risque d'infection devrait être évité autant que possible ; ou lorsque le risque d'attraper la grippe est prédit comme étant faible, il est déterminé que la nécessité d'administrer un vaccin contre la grippe est faible.
